# EUROPÄISCHE PATENTSCHRIFT

(11) **EP 3 242 693 B1**
(45) Veröffentlichungstag und Bekanntmachung des Hinweises auf die Patenterteilung: **14.08.2019**
(21) Anmeldenummer: 16700942.2
(22) Anmeldetag: 07.01.2016
(51) Int. Cl.: A61M 1/16, A61M 1/26, A61M 1/10

(54) **VORRICHTUNG FÜR DEN STOFFAUSTAUSCH ZWISCHEN BLUT UND EINEM GAS/GASGEMISCH**
DEVICE FOR EXCHANGING MATERIALS BETWEEN BLOOD AND A GAS/GAS MIXTURE
DISPOSITIF DE TRANSFERT DE MATIÈRE ENTRE LE SANG ET UN MÉLANGE GAZ/GAS

(30) Priorität: 07.01.2015 DE 102015000021
(43) Veröffentlichungstag der Anmeldung: 15.11.2017
(73) Patentinhaber: enmodes GmbH, 52070 Aachen (DE)
(72) Erfinder: BORCHARDT, Ralf, 52066 Aachen (DE); KAUFMANN, Tim, 52070 Aachen (DE)
(74) Vertreter: Cohausz Hannig Borkowski Wißgott
(86) Internationale Anmeldenummer: PCT/EP2016/000008
(87) Internationale Veröffentlichungsnummer: WO 2016/110446

(56) Entgegenhaltungen:
- US-A1- 2002 143 397
- US-A1- 2005 281 705
- US-A1- 2010 106 072
- US-A1- 2014 061 116

## Beschreibung

Die Erfindung betrifft eine Vorrichtung für den Stoffaustausch zwischen Blut und einem Gas/Gasgemisch, umfassend eine blutdurchströmbare Kammer, in der eine Vielzahl stoffpermeabler Hohlfasern angeordnet ist, wobei die Hohlfasern von dem Gas/Gasgemisch durchströmbar und von Blut umströmbar sind.

Vorrichtungen dieser Art sind im Stand der Technik bekannt und werden oftmals als Oxygenator bezeichnet, wobei diese Vorrichtungen dazu dienen, durch einen Stoffaustausch, insbesondere Gasaustausch über die permeablen Wände der Hohlfasern den CO₂-Partialdruck im Blut abzusenken und den O₂-Partialdruck anzuheben. Zu diesem Zweck lässt man ein Gas oder Gasgemisch durch die Hohlfasern strömen, in welchem der CO₂-Partialdruck geringer ist als im Blut und der O₂-Partialdruck größer, so dass es durch einen Stoffaustausch aufgrund von Diffusion der Gasmoleküle zu einem Ausgleich der Partialdrücke und somit zu einer Sauerstoffanreicherung und CO₂-Abreicherung im Blut kommt.

Solche Oxygenatoren bzw. Vorrichtungen der vorgenannten Art können als künstliche Lunge zum Einsatz kommen, wobei gemäß dem bisherigen Stand der Technik im Wesentlichen solche Vorrichtungen extrakorporal Verwendung finden.

Problematisch ist es beim Einsatz solcher Vorrichtungen insbesondere dann, wenn diese Vorrichtungen vorgesehen werden, um die Funktion der Lunge vollständig zu übernehmen, beispielsweise bei Patienten, die auf eine Lungentransplantation warten. Problematisch ist dies insoweit, als dass durch die Flexibilität der Lungengefäße und insbesondere der Pulmonalarterie ein durch diese Organe oder Organbereiche bereitgestellter sogenannter Windkesseleffekt, nämlich eine elastische Nachgiebigkeit der Gefäße bei Blutdruckschwankungen aufgrund des Herzschlages entfällt und insoweit das Herz bei Einsatz starrer Vorrichtungen für den Stoffaustausch gegen einen erhöhten Widerstand anpumpen muss, was medizinisch bedenklich ist.

Vor diesem Hintergrund wurden bereits Vorrichtungen entwickelt, bei denen das äußere Gehäuse eine flexible Wandung aufweist, die gegenüber dem pulsierenden Blutdruck nachgiebig ist, insoweit also sich das Gehäusevolumen bei erhöhtem Blutdruck dem Blutdruck folgend erhöht und die gesamte Vorrichtung sich vergrößert in den äußeren Dimensionen. Bisherige Vorrichtungen der eingangs genannten Art sind vergleichsweise groß bauend und wenig geeignet für einen bevorzugt intrakorporalen Einsatz,

Relevanter Stand der Technik ist in Dokumenten US2010/0106072 A1, US2014/0061116 A1, US2002/0143397 A1 und US2005/0281705 A1 offenbart.

Es ist daher eine Aufgabe der Erfindung eine Vorrichtung der eingangs genannten Art bereitzustellen und zu verbessern, die gegenüber dem Stand der Technik kleinbauender ist, eine gleichmäßige Blutströmung bereitstellt, extrakorporal und bevorzugt auch intrakorporal eingesetzt werden kann und bevorzugt der rechte Ventrikel des Herzens als Pumpe zur Förderung von Blut durch die Vorrichtung genutzt werden kann, insbesondere ohne dass das Herz über das natürliche Maß hinaus belastet wird.

Eine Vorrichtung gemäß der Erfindung ist in Anspruch 1 definiert. Die Aufgabe wird gemäß der Erfindung dadurch gelöst, dass in der eingangs genannten Kammer zusätzlich zu den vom Gas bzw. Gasgemisch durchströmbaren bzw. im Einsatz durchströmten Hohlfasern wenigstens ein verformbares Element angeordnet ist, welches durch von außen auf das wenigstens eine Element einwirkende, durch Druckschwankungen erzeugte Kräfte, insbesondere durch vom Blut in der Kammer übertragene Druckschwankungen verformbar und rückstellbar ist, insbesondere aus einer relaxierten Form komprimierbar ist und in die relaxierte Form rückstellbar ist.

Anders als bei der natürlichen Windkesselfunktion der Blutgefäße, bei welcher der Blutdruck von innen auf die Gefäßwände wirkt und diese bei Druckerhöhung expandiert, wirkt bei der Erfindung der Blutdruck von außen auf das wenigstens eine verformbare Element, da dieses ebenso wie das Blut in der Kammer angeordnet und vom Blut umgeben ist. Bei sich erhöhendem Blutdruck (z.B. während der Systole bei natürlicher Herztätigkeit oder hervorgerufen durch eine separate pulsatile Blutpumpe) wird daher das wenigstens eine verformbare Element komprimiert, insbesondere aus einer relaxierten Form heraus und bei nachlassendem Blutdruck (z.B. während der Diastole bei natürlicher Herztätigkeit oder hervorgerufen durch eine separate pulsatile Blutpumpe) expandiert das wenigstens eine Element wieder, d.h. wird bevorzugt in die vorherige, insbesondere relaxierte Form zurückgestellt.

Ein erster wesentlicher Vorteil einer solchen Vorrichtung liegt bereits darin, dass durch Druckschwankungen im Blut, die durch den Herzschlag hervorgerufen werden, keine nach außen hin übertragene Volumenvergrößerung der gesamten Vorrichtung einhergeht, sondern vielmehr durch das in der Kammer integrierte wenigstens eine verformbare Element die vom Herzschlag erzeugten Druckschwankungen intern in der Kammer abgefangen werden, wobei in bevorzugter Ausführung das verformbare Element komprimierbar ist, also unter einem sich erhöhenden Blutdruck das Volumen verringert und bei sich verringerndem Blutdruck in eine relaxierte volumenvergrößerte Form zurückstellbar ist. Die Relaxation erfolgt erfindungsgemäß alleine durch interne Rückstellkräfte, wenn der Blutdruck geringer wird, so dass ein solches Element als passiv zu bezeichnen ist, denn es enthält und benötigt keinerlei künstliche Aktorik, um die Herzschlag-synchrone Verformung zu bewirken.

Die Erfindung kann dabei bevorzugt vorsehen, dass es die einzige Funktion des wenigstens einen verformbaren Elementes in der Vorrichtung ist, den Windkesseleffekt bereitzustellen.

Gemäß der Erfindung kann es vorgesehen sein, zumindest ein solches verformbares Element vorzusehen, wobei gemäß bevorzugter Ausführung eine erfindungsgemäße Vorrichtung mehrere solcher verformbarer Elemente im Inneren der Kammer aufweisen kann.

Ein solches verformbares Element kann in einer nicht beanspruchten einfachsten Ausführung beispielsweise ein elastisch verformbares Element aus einem elastischen Vollmaterial sein, wie Beispielsweise einem elastomeren Kunststoff, der nach Kompression aufgrund von außen einwirkender Kräfte reversibel reagiert. Zum Beispiel kann es sich um ein Vulkanisat von Natur- oder Silikonkautschuk handeln.

Insbesondere wird für ein solches verformbares Element ein elastisch verformbarer Kunststoff zum Einsatz kommen, dessen Glasübergangspunkt unterhalb der Einsatztemperatur des Elementes in der erfindungsgemäßen Vorrichtung liegt. Diese Einsatztemperatur ist bei intrakorporalem Einsatz beispielsweise die Körpertemperatur von etwa 37°C bzw. bei extrakorporalem Einsatz die Umgebungstemperatur in Körpernähe, insbesondere im Bereich von 15°C bis 30°C. Bevorzugt kann ein elastisches Material Verwendung finden, welches ein Elastizitätsmodul kleiner 0,05 kN/mm² aufweist.

In der beanspruchten Ausführungsform ist es vorgesehen, dass das wenigstens eine verformbare Element als ein Hohlkörper ausgebildet ist. Ein solcher Hohlkörper kann in seinen äußeren Wandbereichen aus einem verformbaren Material gemäß den vorgenannten Angaben ausgebildet sein, insbesondere aus dem genannten elastischem Material, insbesondere Kunststoff, bevorzugt mit dem genannten Elastizitätsmodul kleiner 0,05 kN/mm².

Ein solcher Hohlkörper, der in besonders bevorzugter Ausführungsform als faserförmiger oder rohrförmiger Hohlkörper, insbesondere somit als ein Hohlkörper mit einer Längserstreckungsrichtung ausgebildet sein kann, ist bevorzugt mit einem nicht durch den Hohlkörper hindurchfließenden Fluid, bevorzugt einem gasförmigen Fluid gefüllt. Eine Füllung mit einem flüssigen Fluid kann ebenso vorgesehen sein. Der Querschnitt eines solchen faser- oder rohrförmigen Hohlkörpers senkrecht zur Längserstreckungsrichtung ist grundsätzlich beliebig, jedoch bevorzugt kreisrund.

Ein solches, bevorzugt gasförmiges Fluid bildet eine komprimierbare Masse in Verbindung mit der Verformbarkeit der Wandbereiche eines solchen Hohlkörpers, insbesondere also der Elastizität eines Hohlkörpers, dessen Wandungsbereiche aus einem elastischen Material, insbesondere Kunststoff ausgebildet sind.

Bevorzugt werden unter einem elastischen Material in Sinne der Erfindung solche Materialien verstanden, die geeignet sind unter der Wirkung von üblichem Blutdruck (bis max. 200 mmHg) derart nachzugeben, dass eine der natürlichen Windkessel-Funktion gleichwertige Funktion erreicht wird, insbesondere eine Volumenvariabilität zwischen Systole und Diastole von 20 bis 80 ml.

Beim Einsatz faserförmiger/rohrförmiger Hohlkörper kann es die Erfindung in bevorzugter Ausführung vorsehen, dass in der Kammer eine Vielzahl von faserförmigen/rohrförmigen, permeablen oder bevorzugt nicht-permeablen Hohlkörpern angeordnet ist und diese Hohlkörper in ihrer Längserstreckungsrichtung parallel zu den stoffpermeablen Hohlfasern liegen, durch welche der Gasaustausch erfolgt und von diesen umgeben sind.
Im gesamten Volumen der Kammer können diese faserförmigen/rohrförmigen Hohlkörper bevorzugt gleichmäßig verteilt angeordnet sein.

Beispielsweise können die faserförmigen oder auch rohrförmigen Hohlkörper, die ein unter Blutdruckeinwirkung komprimierbares Volumen darstellen, von den stoffpermeablen Hohlfasern kontaktierend umgeben sein, d.h. benachbarte permeable Hohlfasern liegen an den faserförmigen/rohrförmigen komprimierbaren Hohlkörpern an, zumindest wenn diese relaxiert sind.

Die verformbaren, bevorzugt faserförmigen Hohlkörper, können in beispielhafter Ausführung in den Wandbereichen aus elastischem Silikon gebildet sein, und somit Silikonfasern darstellen, insbesondere mit dem bereits eingangs genannten Elastizitätsmodul.

Besonders bei der Anordnung einer Vielzahl von verformbaren faserförmigen Hohlkörpern, die zwischen den gaspermeablen Hohlfasern, bevorzugt parallel zu diesen angeordnet sind, wird neben der reinen Volumenänderung zwischen Systole und Diastole auch erreicht, dass während der Systole der Strömungswiderstand für das Blut in der Kammer reduziert wird, da aufgrund der Komprimierung der Hohlkörper in der Systole der freie Strömungsquerschnitt zwischen den Hohlkörpern und Hohlfasern vergrößert wird.

Gemäß der Erfindung ist es vorgesehen, dass durch den wenigstens einen verformbaren Hohlkörper, insbesondere die Vielzahl faserförmiger oder rohrförmiger verformbarer Hohlkörper kein Fluss von bevorzugt gasförmigem Fluid hindurch stattfindet, sondern allenfalls dass ein solcher verformbarer Hohlkörper eine Fluidblase, bevorzugt Gasblase umschließt, so dass lediglich durch einen bevorzugt nur einen einzigen Mündungsbereich eines jeweiligen Hohlkörpers Fluid, bevorzugt Gas in diesen hinein und aus diesem hinaus fließen kann.

Eine Ausführungsform kann jedoch auch vorsehen, dass ein jeweiliger verformbarer Hohlkörper innerhalb der Kammer ein allseitig umschlossenes Fluidvolumen, insbesondere Gasvolumen oder Flüssigkeitsvolumen umgrenzt, also in allen Richtungen geschlossen ist und keine fluidische Verbindung zu einem Bereich außerhalb der Kammer hat.

Eine bevorzugte Ausführung kann vorsehen, dass der wenigstens eine verformbare Hohlkörper insbesondere eine Vielzahl faserförmiger oder rohrförmiger verformbarer Hohlkörper ebenso wie die stoff- bzw. gaspermeablen Hohlfasern in einen Gaseinlassbereich oder einen Gasauslassbereich münden/mündet, der durch ein am Stoffaustausch teilnehmendes Gas/Gasgemisch beaufschlagt oder zumindest beaufschlagbar ist. Bevorzugt erstrecken sich die faserförmigen Hohlkörper wie auch die Hohlfasern für den Gasaustausch zwischen einem Gaseinlassbereich und einem Gasauslassbereich, wobei diese Hohlkörper erfindungsgemäß bevorzugt zur Seite des Gaseinlasses verschlossen sind und in Richtung zum Gasauslass offen, also in diesen münden.

Insbesondere bei einem kontinuierlichen Gasstrom der zwecks Stoffaustausch in einer solchen Vorrichtung vorgesehen ist, werden demnach die faserförmigen Hohlkörper bzw. der wenigstens eine verformbare Hohlkörper mit einem statischen Druck beaufschlagt, der die Verformbarkeit beeinflusst. So kann beispielsweise durch die Fließgeschwindigkeit der statische Druck an oder in einem jeweiligen Hohlkörper beeinflusst werden.

Allgemein sieht die Erfindung vor, dass der/die für die Bereitstellung des Windkesseleffektes wenigstens eine Hohlkörper bzw. die vorgesehenen faser- oder rohrförmigen Hohlkörper im Inneren ausschließlich durch einen statischen Fluid-, bevorzugt Gas-Druck beaufschlagt sind, insbesondere der allenfalls zum Zweck der Änderung auf einen anderen statischen Druck geändert werden kann. Der Druck in den Hohlkörpern variiert somit durch den von außen einwirkenden Blutdruck um den inneren statischen Druck herum, der bei nicht gegebenem Blutdruck vorliegt, insbesondere also z.B. bevor die Vorrichtung als Oxygenator zum Einsatz kommt und mit Blut gefüllt wird. Als relaxierte Form wird dabei bevorzugt diejenige verstanden, die bei dem wenigstens einen Hohlkörper unter Wirkung des diastolischen Blutdruckes vorliegt, insbesondere unter Wirkung eines innen in den Hohlkörpern vorherrschenden kontinuierlich statischen Druckes von Gas.

Besonders in einer solchen Ausführung kann es auch vorgesehen sein, dass die faserförmigen oder rohrförmiger Hohlkörper aus einem gaspermeablen Material ausgebildet sind, so dass ein Gasaustausch grundsätzlich auch durch diese Hohlkörper stattfinden kann, wenngleich ein Gaswechsel nicht durch einen Gasdurchfluss innerhalb dieser Hohlkörper stattfindet. Hingegen werden die Hohlkörper pulsierend komprimiert und relaxiert, wodurch ein Gaswechsel ebenso stattfinden kann. In dieser Ausführung stellen zwar die Hohlkörper nicht ausschließlich nur die Windkesselfunktion bereit, wie es eingangs genannt wurde, sondern nehmen auch am Gasaustauch teil, weisen jedoch weiterhin eine Passivität auf, d.h. werden nur durch den natürlichen Blutdruck von außen periodisch druckbeaufschlagt. Eine innere periodische Druckbeaufschlagung erfolgt auch hier weiterhin nicht.

Eine andere Ausführung kann auch vorsehen, dass der wenigstens eine verformbare Hohlkörper, insbesondere eine Vielzahl faserförmiger oder rohrförmiger verformbarer Hohlkörper in einen zur Kammer separaten Raum mündet/n, der mit einem gasförmigen oder flüssigen Fluid füllbar ist. Hier kann es z.B. vorgesehen sein, dass im Raum ein Gas oder eine Flüssigkeit verwendet wird, das/die am Stoffaustausch nicht teilnimmt.

Besonders in dieser Ausführung kann es vorgesehen sein, dass die verformbaren Hohlkörper, die im Wesentlichen lediglich eingesetzt sind, um einen WindkesselEffekt bereitzustellen, nicht permeabel sind hinsichtlich eines Stoffaustausches, so dass sichergestellt ist, dass keine Möglichkeit besteht, dass das in den Hohlkörpern eingesetzte Gas oder Gasgemisch oder die Flüssigkeit selbst am Stoffaustausch teilnimmt.

Sofern der genannte separate Raum hingegen mit einem Gas oder Gasgemisch beaufschlagt ist, welches demjenigen Gas oder Gasgemisch entspricht, das für den Stoffaustausch zum Einsatz kommt, kann grundsätzlich auch als verformbarer Hohlkörper ein solcher zum Einsatz kommen, der eine Stoffpermeabilität insbesondere für O₂ und CO₂ aufweist.

Durch die Konstruktion, dass eine Vielzahl verformbarer Hohlkörper in einen separaten Raum einmündet, kann sichergestellt werden, dass das zur Komprimierung unter dem schwankenden Blutdruck zur Verfügung stehende Volumen insgesamt größer ist als das von den Hohlkörpern selbst in der Kammer umschlossene Volumen, nämlich um den Volumenbetrag der von dem separaten Raum gebildet wird.

In konstruktiv bevorzugter Ausführung kann es die Erfindung vorsehen, dass dieser genannte wenigstens eine Raum betrachtet in der axialen Strömungsrichtung, die beim Gas in der Kammer durch die gas-permeablen Hohlfasern vorliegt, hinter dem Gasauslass angeordnet ist. Hierfür können die stoff- bzw. gas-permeabalen Hohlfasern in den Gasauslass münden, der z.B. ring- oder scheibenförmig um die Mittenachse herum angeordnet ist, wohingegen die den Windkesseleffekt bereitstellenden faser- oder rohrförmigen Hohlkörper durch den Gasauslass hindurchgeführt sind, bevorzugt in derselben axialen Erstreckung, die in der Kammer vorliegt und sodann erst in den axial nachfolgend angeordneten Raum einmünden.

Des Weiteren kann eine erfindungsgemäße Weiterbildung auch vorsehen, dass der Fluiddruck, bevorzugt Gasdruck in dem separaten Raum variabel, insbesondere variabel statisch eingestellt werden kann, z.B. durch einen externen Anschluss über welchen der interne Druck in dem Raum und somit auch in den Hohlkörpern geändert werden kann. So besteht die Möglichkeit den Widerstand zu verändern, den die verformbaren Hohlkörper, insbesondere die Vielzahl verformbarer faserförmiger Hohlkörper dem Blutdruck und der dadurch auf die Hohlkörper ausgeübten Kraft entgegenbringen.

Die Erfindung kann in einer Weiterbildung auch vorsehen, dass nicht nur ein einziger separater fluidfüllbarer Raum vorgesehen ist, in den die Hohlkörper münden, sondern dass beispielsweise wenigstens zwei separate Räume vorgesehen sind, wobei in den einen separaten Raum eine erste Anzahl von faserförmigen/rohrförmigen verformbaren Hohlkörpern münden und in den zweiten Raum eine zweite Anzahl von anderen faserförmigen verformbaren Hohlkörpern.

Gleiches kann gelten, wenn an dem jeweiligen Raum jeweils nur ein einziger verformbarer Hohlkörper zugeordnet ist.

So besteht die Möglichkeit, diese zwei Gruppen von Anzahlen von verformbaren Hohlkörpern intern mit unterschiedlichen Drücken zu beaufschlagen und somit der Formveränderung der Hohlkörper gezielter auf individuell notwendige Bedürfnisse anzupassen.

Konstruktiv wird es als vorteilhaft angesehen, dass der wenigstens eine separate Raum an einem der beiden axialen Enden der Vorrichtung angeordnet ist.

Als axiale Enden der Vorrichtung werden diejenigen verstanden, die in Richtung der Längserstreckungsrichtungen der permeablen Hohlfasern und/oder der faserförmigen / rohrförmigen Hohlkörper beabstandet sind. Senkrecht zu dieser axialen Erstreckungsrichtung kann die Vorrichtung z.B. kreisförmigen oder mehreckigen Querschnitt aufweisen.

Der wenigstens eine genannte separate Raum kann hier insbesondere an demjenigen axialen Ende angeordnet sein, welches demjenigen anderen Ende gegenüberliegt, an welchem sowohl Bluteinlass als auch Blutauslass angeordnet sind.

Beispielsweise kann sowohl bei der vorgenannten Ausführung als auch bei allen anderen denkbaren Ausführungen der erfindungsgemäßen Vorrichtung die Konstruktion derart gewählt sein, dass an einem der beiden axialen Enden einer erfindungsgemäßen Vorrichtung ein Bluteinlass und ein Blutauslass angeordnet ist. Beispielsweise kann der Bluteinlass in einen die Kammer umgebenden, ringförmigen Umfangsbereich münden, so dass hier die Möglichkeit besteht, dass aus diesem Umfangsbereich das Blut in radialer Richtung bezogen auf die Mittenachse der Kammer in die Kammer einströmen kann und wobei weiterhin in der Kammer ein Kanal angeordnet ist, der in den Blutauslass der Vorrichtung mündet, der bevorzugt koaxial zur Mittenachse der Vorrichtung liegt und der von den stoffpermeablen Hohlfasern und mehreren faserförmigen/rohrförmigen Hohlkörpern umgeben ist.

Dieser Kanal weist bevorzugt nahe dem anderen Ende, welches also dem Bluteinlass und Blutauslass gegenüberliegt, wenigstens eine Öffnung auf, durch welche Blut aus dem Kammerinneren in den Kanal übertreten kann. So wird durch diese Anordnung erzielt, dass das Blut derart in der Vorrichtung strömt, dass es zum einen von radial außen in die Richtung radial nach innen strömt und hierbei gleichzeitig von demjenigen Ende an welchem es in die Kammer einströmt, zunächst zum gegenüberliegenden Ende strömt, um sodann innerhalb der Kammer wieder zum Auslass am anderen Ende zurück zu gelangen.

Der Blutfluß kann bei gleicher Konstruktion grundsätzlich auch in umgekehrter Richtung erfolgen, es sind also nur Bluteinlass und -auslass vertauscht, insbesondere nur funktional jedoch nicht konstruktiv.

Gaseinlass und Gasauslass können in axialer Erstreckungsrichtung der Vorrichtung gegenüberliegend an den beiden axialen Enden konstruktiv realisiert sein, wobei es vorgesehen sein kann, dass Gas im Gegenstrom oder Gleichstrom zum Blut fließen zu lassen.

Bevorzugte Ausführungsformen der Erfindung werden anhand der nachfolgenden Figuren näher erläutert.

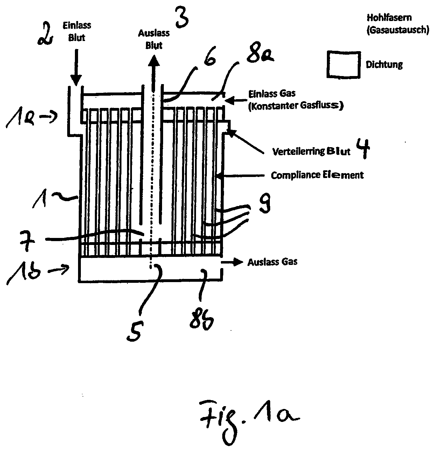

Die Figur 1a zeigt eine Ausführung, die eine Längserstreckungsrichtung mit Bezug auf die Figur 1a von oben nach unten, d.h. in der Papierebene aufweist, wobei senkrecht zur Papierebene die Querschnittsform der Vorrichtung beispielsweise rund ist. Jede andere Querschnittsform ist ebenso möglich.

Die Vorrichtung hat ein Gehäuse, welches eine Kammer 1 umschließt, in welcher Blut vom Bluteinlass 2 zum Blutauslass 3 strömen kann. Hierbei ist der Bluteinlass derart aufgebaut, dass zwar in axialer Richtung Blut in den Einlass einströmen kann, jedoch durch einen ringförmigen Bereich 4 der die gebildete Kammer 1 an einem axialen Ende umgibt, in die äußere Peripherie der Kammer 1 an diesem axialen Ende umgeleitet wird, um sodann von diesem ringförmigen Bereich 4 in radialer Richtung nach innen in die Kammer 1 einzuströmen.

Bezogen auf die Längserstreckungsrichtung und die Mittenachse 5 der Kammer 1 ist koaxial zu dieser in der Vorrichtung bzw. in dem von dem Gehäuse umschlossenen Kammervolumen ein Kanal 6 angeordnet, dessen hier oberes Ende in den Blutauslass 3 mündet und der ein in der Kammer 1 liegendes Ende aufweist, das nahe dem anderen axialen Ende wenigstens eine Öffnung 7 aufweist, in die das Blut aus der Kammer 1 in den Kanal 6 übertreten kann. So gelangt das Blut auf seinem Fließweg von dem oberen Ende 1a des Gehäuses auf seinem Weg radial von außen nach innen in Richtung zum unteren Ende 1b der Vorrichtung, um sodann durch den Kanal 6 zum Blutauslass 3 zurückzuströmen.

An den beiden axialen Enden 1a und 1b des Gehäuses sind Ein- und Auslassbereiche 8a und 8b für am Stoffaustausch teilnehmendes Gas angeordnet, wobei der Einlassbereich 8a von dem Gas angeströmt ist, das für den Stoffaustausch vorgesehen ist. Der Querschnitt von Gaseinlass 8a und -auslass 8b ist jeweils so groß, dass der gesamte Kammerquerschnitt senkrecht zur axialen Erstreckung überdeckt ist.

Das Gas strömt von dem Einlassbereich 8a durch eine Vielzahl von in den Gaseinlassbereich 8a einmündenden permeablen Hohlfasern, die für den Stoffaustausch eingesetzt werden, in Richtung zum Auslassbereich 8b, in welche die Hohlfasern am anderen Ende ebenfalls einmünden, um von dort zum Gasauslass zu gelangen.

In der Figur 1a ist die Vielzahl der am Stoffaustausch teilnehmenden permeablen Hohlfasern zwecks Bewahrung der Übersichtlichkeit nicht eingezeichnet. Diese Hohlfasern erstrecken sich jedoch jeweils parallel zur axialen Richtung bzw. der Mittenachse 5 zwischen dem Ein- und Auslassbereich 8a und 8b und münden jeweils in diese, so dass die inneren Volumina der am Stoffaustausch teilnehmenden Hohlfasern mit dem Gasauslass und dem Gaseinlass kommunizieren.

Gemäß der Erfindung ist es hier vorgesehen, dass in einer Erstreckung parallel zu der Mittenachse 5 bzw. der Vielzahl der am Stoffaustausch teilnehmenden Hohlfasern zusätzliche faserförmige oder rohrförmige Hohlkörper 9 eingesetzt sind, die hier den Kanal 6 ebenso in paralleler Anordnung umgeben. Das interne Kammervolumen ist somit neben dem Blut gefüllt durch sowohl die am Stoffaustausch teilnehmenden Hohlfasern als auch die, insbesondere nicht am Stoffaustausch teilnehmenden faserförmigen Hohlkörper, die gemäß der Erfindung verformbare insbesondere elastische Außenwandbereiche aufweisen und somit beispielsweise aus Silikonhohlfasern bzw. hohlen Silikonschläuchen oder Schläuchen bzw. Fasern aus anderen elastischen Materialien ausgebildet sind, oder aus anderen elastischen Materialien, insbesondere mit einem Elastizitätsmodul kleiner 0,05 kN/mm².

In der Figur 1a ist es erkennbar, dass jeder der verformbaren Hohlkörper 9 an seinem oberen Ende einen offenen Mündungsbereich hat, der in den Gaseinlassbereich 8a mündet, wohingegen der untere Bereich, der nahe dem Gasauslassbereich 8b liegt, verschlossen ist. Jeder Hohlkörper 9 erstreckt sich dabei hier bevorzugt durch die gesamte Kammer 1 hindurch. Hierdurch wird bewirkt, dass zwar das Innere der verformbaren Hohlkörper 9 durch das am Stoffaustausch beteiligte Gas beaufschlagt ist, das Gas jedoch nicht durch die Hohlkörper hindurchfließen kann.

Die verformbaren Hohlkörper 9 bilden in ihrer Vielzahl ein insgesamt komprimierbares Volumen, das sich unter einwirkendem Blutdruck bei Blutdruckerhöhung (z.B. bei der natürlichen Systole oder durch eine pulsatile Blutpumpe) verkleinert und unter wirkenden verringerten Blutdruck (z.B. bei der natürlichen Diastole oder durch eine pulsatile Blutpumpe) wieder in seine relaxierte Ursprungsform zurückerweitert. So kann dieses Volumen durch den Herzschlag hervorgerufene Blutdruckschwankungen aufnehmen und darüber hinaus maßgeblich passiv durch die Verformbarkeit zum Pumpeffekt des Herzens beitragen, weil bei der Relaxation Blut aus der Kammer verdrängt wird.

Die Vielzahl der verformbaren Hohlkörper 9 kann somit zur Ausbildung eines Windkessel-Effektes beitragen, der ansonsten durch die Flexibilität von Gefäßgewebe, insbesondere der Pulmonalarterie erzielt wird und das Herz in seiner Arbeit maßgeblich entlastet sowie die Pumparbeit unterstützt.

Besonders bei einem vollständigen Ersatz einer Lungenfunktion durch eine solche erfindungsgemäße Vorrichtung kann die Vorrichtung somit auf passive Art und Weise, d.h. ohne dass die verformbaren Hohlfasern in irgendeiner Art aktiv künstlich angesteuert würden, zu einer Herzentlastung beitragen.

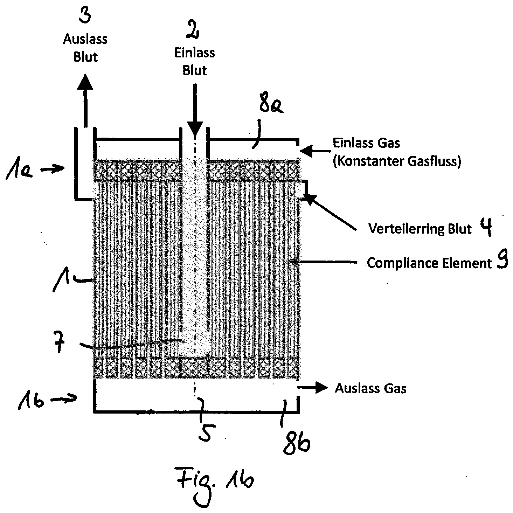

Figur 1b zeigt eine Variante der Figur 1a, in der Bluteinlass 2 und Blutauslass 3 lediglich funktional vertauscht sind, wobei jedoch die Hohlkörper 9, die den Windkesseleffekt bereitstellen, hier gaseinlassseitig permanent, insbesondere auch nicht öffenbar verschlossen sind und stattdessen in den Gasauslass 8b münden. Hier sind weiterhin die für den Gasaustausch zuständigen Hohlfasern zwischen den Hohlkörpern 9 als Striche visualisiert.

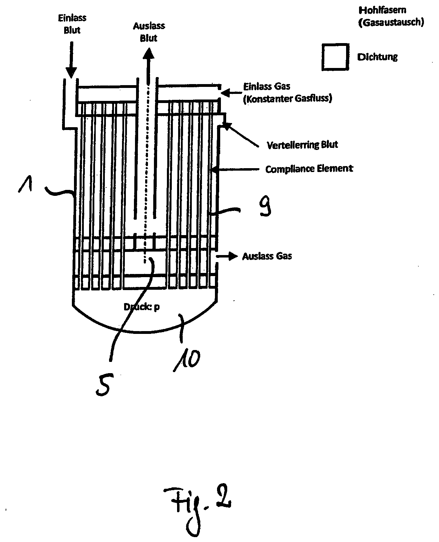

Die Figur 2 zeigt eine andere mögliche Ausgestaltungsvariante, bei der die erfindungsgemäße Vorrichtung grundsätzlich gleiche Konstruktion aufweist, d. h. dass das Gehäuse eine Kammer 1 umschließt, welche von Blut durchströmt ist, auf genau die gleiche Art und Weise, wie dies bei den Figuren 1 hinsichtlich der Strömungsführung realisiert ist. Die diesbezüglichen Konstruktionsmerkmale gelten hier ebenso.

Hier ist die Ausgestaltung hingegen derart, dass die einzelnen verformbaren Hohlkörper 9, die auch hier faserförmig oder rohrförmig ausgebildet sind und parallel zu den stoffpermeablen Hohlfasern und parallel zur Mittenachse 5 ausgerichtet sind, mit ihrem jeweils offenen und hier unteren Ende in einen gemeinsamen zur Kammer und zur Umgebung separaten Raum 10 münden, so dass das insgesamt zur Verfügung stehende komprimierbare Volumen gegeben ist durch die Summe der einzelnen Volumina jedes einzelnen verformbaren Hohlkörpers 9 sowie dem Volumen des Raumes 10.

Hier kann die Möglichkeit bestehen, den Raum 10 individuell mit einem bestimmten gewünschten Druck zu beaufschlagen, um so die Steifigkeit bzw. Elastizität und Komprimierbarkeit des Gesamtvolumens einstellen zu können.

Die Gesamtanordnung aus dem insgesamt gebildeten Volumen und der Vielzahl aus einem bevorzugt elastischen Material ausgebildeten verformbaren Hohlkörpern 9 bildet somit eine Art Federelement, dessen Federkonstante durch den Druck individuell geändert werden kann. So besteht die Möglichkeit, die Verformung und die Volumenänderung der einzelnen Hohlkörper 9 bei den bestehenden Blutdruckschwankungen, die durch den Herzschlag hervorgerufen werden, anwendungsspezifisch zu ändern.

Beispielsweise kann es hierfür vorgesehen sein, dass an dem separaten Raum 10 ein Anschluss zur Zuführung oder zum Ablassen von Gas vorgesehen ist, um den internen Druck zu ändern, was die Figur 2 vorliegend nicht visualisiert.

Statt hier nur einen einzigen separaten Raum 10 an dem einen hier unteren axialen Ende der Vorrichtung anzuordnen, kann die Erfindung auch vorsehen, zwei oder mehr separate Räume vorzusehen, in die jeweils eine bestimmte Anzahl der insgesamt zur Verfügung stehenden Hohlkörper 9 münden. So kann bei diesen so gebildeten verschiedenen Gruppen von verformbaren Hohlkörpern 9 deren elastische Reaktion auf Blutdruckänderungen unterschiedlich eingestellt werden.

Beide Ausführungsvarianten gestatten beispielsweise ein intrakorporalen Einsatz der Vorrichtung, insbesondere um eine Lungenfunktion komplett zu ersetzen oder aber um parallel zur natürlichen Lunge betrieben zu werden, wobei durch die interne Verformbarkeit der Vielzahl von Hohlkörpern 9 das Herz in seiner Arbeit maßgeblich und insbesondere variabel einstellbar entlastet werden kann. Daher kann das Herz selbst und insbesondere der rechte Ventrikel als Pumpe zum Betrieb der Vorrichtung genutzt werden.

Der Raum 10 kann zur äußeren Umgebung hin wenigstens eine flexible Wandung, z.B. aus einem Elastomer (beispielsweise ein Silikon) aufweisen oder alternativ starr ausgebildet sein.

So wie es zwischen Figuren 1a und 1b visualisiert ist können auch hier Bluteinlass und -auslass funktional vertauscht werden, insbesondere ohne die Konstruktion zu ändern.

## Patentansprüche

1. Vorrichtung für den Stoffaustausch zwischen Blut und einem Gas/Gasgemisch, umfassend eine blutdurchströmbare Kammer (1), in der eine Vielzahl stoffpermeabler Hohlfasern angeordnet ist, wobei die Hohlfasern von dem Gas / Gasgemisch durchströmbar und von Blut umströmbar sind, wobei in der Kammer (1) zusätzlich zu den durchströmbaren Hohlfasern wenigstens ein verformbares, vom Blut umströmbares Element (9) angeordnet ist, welches durch vom Blut in der Kammer (1) von außen auf das wenigstens eine Element (9) einwirkende Druckschwankungen verformbar und rückstellbar ist, **dadurch gekennzeichnet, dass** das wenigstens eine Element (9) als Hohlkörper und passiv ausgebildet ist und eingerichtet ist zur Bereitstellung eines Windkesseleffektes, wofür das wenigstens eine Element im Inneren ausschließlich durch einen statischen Fluid-Druck beaufschlagt ist und während eines Anstiegs des Blutdruckes, aus einer relaxierten Form komprimierbar ist und bei einer Abnahme des Blutdruckes in die relaxierte Form rückstellbar ist.

2. Vorrichtung nach Anspruch 1, **dadurch gekennzeichnet, dass** das wenigstens eine verformbare Element (9), ohne jegliche künstliche Druck-Ansteuerung ausgebildet ist, bevorzugt das wenigstens eine verformbare Element (9) ausschließlich nur zur Bereitstellung des Windkesseleffektes vorgesehen ist.

3. Vorrichtung nach einem der vorherigen Ansprüche, **dadurch gekennzeichnet, dass** das wenigstens eine verformbare Element (9) als Hohlkörper, insbesondere als faserförmiger oder rohrförmiger Hohlkörper (9) ausgebildet ist, welcher mit einem nicht durch den Hohlkörper (9) hindurchfließenden Fluid, insbesondere einem gasförmigen Fluid gefüllt ist, bevorzugt wobei der wenigstens eine Hohlkörper an einem seiner Enden permanent, insbesondere nicht öffenbar, verschlossen ist.

4. Vorrichtung nach Anspruch 2 oder 3, **dadurch gekennzeichnet, dass** in der Kammer (1) eine Vielzahl von faserförmigen oder rohrförmigen bevorzugt nicht-permeablen Hohlkörpern (9) angeordnet ist, insbesondere die durch elastische Silikonfasern gebildet sind, bevorzugt die faserförmigen Hohlkörper (9) in der Längserstreckungsrichtung parallel zu den stoffpermeablen Hohlfasern liegen und von diesen umgeben sind, insbesondere von diesen kontaktierend umgeben sind.

5. Vorrichtung nach Anspruch 3 oder 4, **dadurch gekennzeichnet, dass** der wenigstens eine verformbare Hohlkörper (9), insbesondere eine Vielzahl faserförmiger oder rohrförmiger verformbarer Hohlkörper (9) ebenso wie die stoffpermeablen Hohlfasern in einen Gaseinlassbereich (8a) oder einen Gasauslassbereich (8b) mündet/münden, der durch ein am Stoffaustausch teilnehmendes Gas / Gasgemisch beaufschlagbar ist.

6. Vorrichtung nach einem der vorherigen Ansprüche, **dadurch gekennzeichnet, dass** der wenigstens eine verformbare Hohlkörper, insbesondere eine Vielzahl faserförmiger verformbarer oder rohrförmiger Hohlkörper (9) in einen separaten Raum (10) münden, der mit einem bevorzugt gasförmigen Fluid füllbar ist, insbesondere am Stoffaustausch nicht teilnehmenden Fluid füllbar ist, insbesondere wobei der Raum (10) in axialer Strömungsrichtung des Gases in der Kammer (1) hinter dem Gasauslassbereich (8b) angeordnet ist.

7. Vorrichtung nach Anspruch 6, **dadurch gekennzeichnet, dass** der Fluiddruck, insbesondere Gasdruck in dem separaten Raum (10) variabel statisch einstellbar ist.

8. Vorrichtung nach Anspruch 6, **dadurch gekennzeichnet, dass** der separate Raum (10) an einem der beiden axialen Enden der Vorrichtung angeordnet ist, insbesondere an demjenigen axialen Ende, welches einem Ein- (2) und Auslass (3) für Blut gegenüberliegt.

9. Vorrichtung nach einem der vorherigen Ansprüche, **dadurch gekennzeichnet, dass** an einem der beiden axialen Enden der Vorrichtung je ein Anschluß für einen Bluteinlass (2) und einen Blutauslass (3) angeordnet ist, wobei einer der Anschlüsse, insbesondere der Bluteinlass (2) in einen die Kammer (1) umgebenden, ringförmigen Umfangsbereich (4) mündet, insbesondere aus welchem das Blut in radialer Richtung in die Kammer (1) strömbar ist und in den anderen Anschluß, insbesondere den Blutauslass (3) ein Kanal (6) mündet, der von den stoffpermeablen Hohlfasern und mehreren, insbesondere faserförmigen Hohlkörpern(9) umgeben ist, insbesondere der koaxial zur Mittenachse (5) der Vorrichtung angeordnet ist, und der nahe dem anderen axialen Ende wenigstens eine Öffnung (7) aufweist, insbesondere durch welche Blut aus der Kammer (1) in den Kanal (6) übertreten kann.

## Claims

1. Device for substance exchange between blood and a gas/gas mixture, comprising a chamber (1) through which blood can flow and in which a large number of substance-permeable hollow fibres are arranged, wherein the gas/gas mixture can flow through the hollow fibres and blood can flow around them, wherein the chamber (1) accommodates, in addition to the hollow fibres through which the gas/gas mixture can flow, at least one deformable element (9) around which the blood can flow and which is deformable and restorable by pressure fluctuations acting externally on the at least one element (9) from the blood in the chamber (1), **characterized in that** the at least one element (9) is configured as a hollow body and is passive and designed to make available a Windkessel effect, for which reason the at least one element in the interior is acted upon exclusively by a static fluid pressure and, during an increase of the blood pressure, is compressible from a relaxed shape, and, as the blood pressure decreases, is restorable to the relaxed shape.

2. Device according to Claim 1, **characterized in that** the at least one deformable element (9) is designed without any artificial pressure control, preferably the at least one deformable element (9) is exclusively provided only to make available the Windkessel effect.

3. Device according to either of the preceding claims, **characterized in that** the at least one deformable element (9) is designed as a hollow body, in particular as a fibre-shaped or tubular hollow body (9), which is filled with a fluid, in particular a gaseous fluid, that does not flow through the hollow body (9), preferably wherein the at least one hollow body is closed at one of its ends permanently, in particular so as not to be openable.

4. Device according to Claim 2 or 3, **characterized in that** a large number of fibre-shaped or tubular, preferably non-permeable hollow bodies (9) are arranged in the chamber (1), which hollow bodies (9) are in particular formed by elastic silicone fibres, preferably the fibre-shaped hollow bodies (9) lie in the direction of the longitudinal extent parallel to the substance-permeable hollow fibres and are surrounded by these, in particular are surrounded by and in contact with these.

5. Device according to Claim 3 or 4, **characterized in that** the at least one deformable hollow body (9), in particular a large number of fibre-shaped or tubular deformable hollow bodies (9), likewise the substance-permeable hollow fibres open(s) into a gas inlet region (8a) or a gas outlet region (8b) which can be acted upon by a gas/gas mixture that participates in the substance exchange.

6. Device according to one of the preceding claims, **characterized in that** the at least one deformable hollow body, in particular a large number of fibre-shaped deformable or tubular hollow bodies (9), open(s) into a separate space (10) that is fillable with a preferably gaseous fluid, in particular fillable with a fluid that does not participate in the substance exchange, in particular wherein the space (10) is arranged downstream from the gas outlet region (8b) in the axial direction of flow of the gas in the chamber (1).

7. Device according to Claim 6, **characterized in that** the fluid pressure, in particular the gas pressure, in the separate space (10) is variably and statically adjustable.

8. Device according to Claim 6, **characterized in that** the separate space (10) is arranged at one of the two axial ends of the device, in particular at that axial end lying opposite an inlet (2) and outlet (3) for blood.

9. Device according to one of the preceding claims, **characterized in that** a port for a blood inlet (2) and a port for a blood outlet (3) are arranged at one of the two axial ends of the device, wherein one of the ports, in particular the blood inlet (2), opens into an annular circumferential region (4) which surrounds the chamber (1), in particular from which the blood can flow in the radial direction into the chamber (1), and a channel (6) opens into the other port, in particular the blood outlet (3), and is surrounded by the substance-permeable hollow fibres and by a plurality of in particular fibre-shaped hollow bodies (9), which channel (6) is in particular arranged coaxially with respect to the central axis (5) of the device and, near the other axial end, has at least one opening (7), in particular through which blood can pass from the chamber (1) into the channel (6).

## Revendications

1. Dispositif de transfert de matière entre le sang et un mélange gaz/gaz, comprenant une chambre (1) pouvant être traversée par le sang, dans laquelle sont disposées une multiplicité de fibres creuses perméables aux matières, dans lequel les fibres creuses peuvent être traversées par le mélange gaz/gaz et baignées par le sang, dans lequel au moins un élément déformable (9), pouvant être baigné par le sang, est disposé dans la chambre (1) en plus des fibres creuses pouvant être traversées, qui peut être déformé et restauré par des fluctuations de pression provoquées par le sang dans la chambre (1) par l'extérieur sur ledit au moins un élément (9), **caractérisé en ce que** ledit au moins un élément (9) est formé par un corps creux et est passif, et est conçu pour la production d'un effet Windkessel, pour lequel ledit au moins un élément est soumis à l'intérieur exclusivement à une pression de fluide statique et il peut être comprimé à partir d'une forme relâchée pendant une hausse de la pression sanguine et être restauré dans la forme relâchée lors d'une baisse de la pression sanguine.

2. Dispositif selon la revendication 1, **caractérisé en ce que** ledit au moins un élément déformable (9) est réalisé sans aucune commande de pression artificielle, de préférence ledit au moins un élément déformable (9) n'est prévu exclusivement que pour la production de l'effet Windkessel.

3. Dispositif selon l'une quelconque des revendications précédentes, **caractérisé en ce que** ledit au moins un élément déformable (9) est formé par un corps creux, en particulier par un corps creux en forme de fibre ou en forme de tube (9), qui est rempli avec un fluide ne circulant pas à travers le corps creux (9), en particulier un fluide gazeux, de préférence dans lequel ledit au moins un corps creux est fermé à ses deux extrémités de façon permanente, en particulier d'une façon ne pouvant pas être ouverte.

4. Dispositif selon une revendication 2 ou 3, **caractérisé en ce qu'**une multiplicité de corps creux (9) en forme de fibre ou en forme de tube, de préférence non perméables, sont disposés dans la chambre (1), en particulier qui sont formés par des fibres de silicone élastiques, de préférence les corps creux en forme de fibre (9) sont placés dans la direction d'extension longitudinale parallèlement aux fibres creuses perméables aux matières et sont entourés par celles-ci, en particulier sont entourés par celles-ci avec contact.

5. Dispositif selon une revendication 3 ou 4, **caractérisé en ce que** ledit au moins un corps creux déformable (9), en particulier une multiplicité de corps creux déformables en forme de fibre ou en forme de tube (9) ainsi que les fibres creuses perméables aux matières débouchent dans une zone d'entrée de gaz (8a) ou dans une zone de sortie de gaz (8b), qui peut être soumise au mélange gaz/gaz participant au transfert de matières.

6. Dispositif selon l'une quelconque des revendications précédentes, **caractérisé en ce que** ledit au moins un corps creux déformable, en particulier une multiplicité de corps creux déformables en forme de fibre ou en forme de tube (9), débouchent dans une chambre séparée (10), qui peut être remplie avec un fluide de préférence gazeux, en particulier peut être remplie avec un fluide ne participant pas au transfert de matières, en particulier dans lequel la chambre (10) est disposée dans la direction axiale d'écoulement du gaz dans la chambre (1) derrière la zone de sortie de gaz (8b).

7. Dispositif selon la revendication 6, **caractérisé en ce que** la pression de fluide, en particulier la pression de gaz dans la chambre séparée (10), est réglable de façon statiquement variable.

8. Dispositif selon la revendication 6, **caractérisé en ce que** la chambre séparée (10) est disposée à l'une des deux extrémités axiales du dispositif, en particulier à l'extrémité axiale qui est opposée à une entrée (2) et une sortie (3) pour le sang.

9. Dispositif selon l'une quelconque des revendications précédentes, **caractérisé en ce qu'**un raccord pour une entrée de sang (2) et une sortie de sang (3) est disposé respectivement à une des deux extrémités axiales du dispositif, en particulier l'entrée de sang (2) débouche dans une zone périphérique annulaire (4) entourant la chambre (1), en particulier hors de laquelle le sang peut s'écouler en direction radiale dans la chambre (1) et un canal (6) débouche dans l'autre raccord, en particulier la sortie de sang (3), lequel est entouré par les fibres creuses perméables aux matières et plusieurs corps creux (9) en particulier en forme de fibre, et qui présente à proximité de l'autre extrémité axiale au moins une ouverture (7), en particulier par laquelle du sang peut passer de la chambre (1) dans le canal (6).
